# EUROPEAN PATENT APPLICATION

(11) **EP 4 497 436 A1**
(43) Date of publication of application: **29.01.2025**
(21) Application number: 23774893.4
(22) Date of filing: 20.03.2023
(51) Int. Cl.: A61K 31/497, A61K 31/4545, A61P 25/08, A61P 25/18, A61P 25/28, A61P 43/00

(54) **THERAPEUTIC OR PROPHYLACTIC MEDICINE FOR FRAGILE X SYNDROME**

(30) Priority: 22.03.2022 JP 2022045557
(71) Applicant: Kyoto University, Kyoto-shi, Kyoto 606-8501 (JP)
(72) Inventor: INOUE, Haruhisa, Kyoto-shi, Kyoto 606-8501 (JP); IMAMURA, Keiko, Kyoto-shi, Kyoto 606-8501 (JP)
(74) Representative: Hoffmann Eitle
(86) International application number: PCT/JP2023/010968
(87) International publication number: WO 2023/182298

(57) **Abstract**

The present invention provides a therapeutic or prophylactic medicine for fragile X syndrome containing a compound represented by formula (I) 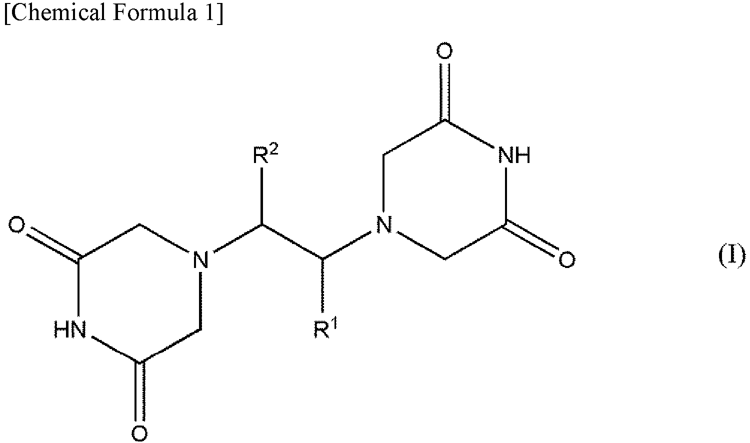 (see the attached description for the symbols in formula (I)) or formula (II) 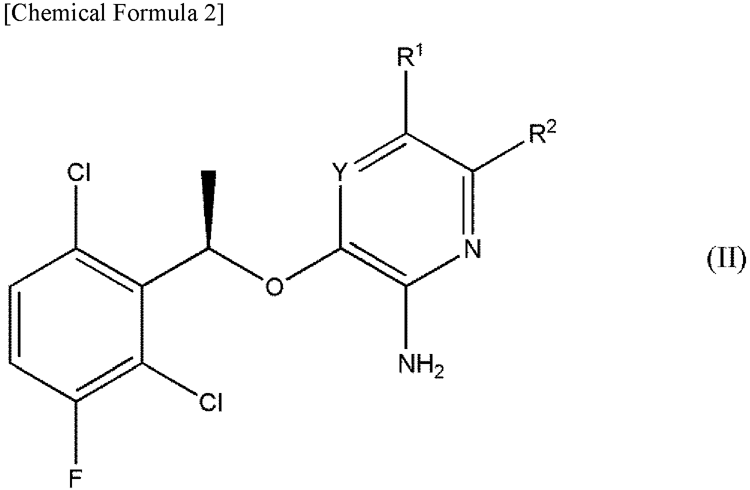 (see the attached description for the symbols in formula (II)) or a salt thereof.

## Description

### FIELD

The present invention relates to a therapeutic or prophylactic medicament for fragile X syndrome. In particular, the present invention relates to a therapeutic or prophylactic medicament for fragile X syndrome, comprising dexrazoxane or an analog thereof or crizotinib or an analog thereof.

### BACKGROUND

Fragile X syndrome (FXS) is a disease characterized by intellectual disability, and one in 3,600 males develops this disease. FXS patients exhibit symptoms of broad autism phenotype, such as intellectual disability, cognitive impairment, and social impairment (e.g., NPL 1). Physical features of FXS patients include a long and narrow face, large auricles, and large testicles. FXS patients often suffer from various psychiatric symptoms after puberty, and about 15% to 20% of male patients have epilepsy. In some cases, FXS patients may also suffer from complications such as joint hypermobility, flat feet, mitral valve prolapse, strabismus, otitis media, and eating disorders due to gastroesophageal reflux disease. These disorders are caused by loss of fragile X mental retardation protein (FMRP) encoded by the FMR1 (fragile X mental retardation 1) gene during brain development. At present, there is no effective fundamental therapeutic medicament for FXS.

FMRP is a neuronal RNA-binding protein known for its role as a molecular brake on local protein synthesis during synaptic development, and thus is essential for maintaining normal synaptic plasticity (e.g., NPL 2). It is known that FMR1-gene knockout mice show a prolonged "UP state" duration, resulting in an increased neural firing rate (NPL 1). Likewise, it is also known that neurons derived from human pluripotent stem cells (iPS cells) show an increased neural firing rate (NPL 3).

FXS patients have expanded CGG codon repeats (typically more than 200 repeats) in the FMR1 gene. Suppression of the FMR1 gene expression is caused by hypermethylation of these repeats and the neighboring CpG island (FMR1 gene promoter region), and this is considered as the core of the pathophysiology of FXS. Also, it has been reported that, even if there are expanded CGG codon repeats, the FMR1 gene expression is restored by removing the hypermethylation (NPL 3).

Meanwhile, drug repositioning (DR) is a new research concept that is being discussed as a method for breaking through the current impasse in research for new drug development. DR is a strategy aimed at discovering new drug efficacies of existing drugs whose safety and pharmacokinetics in humans have already been confirmed by their proven results and putting them into practical application. A large amount of available existing data can reduce the cost required for development, and there are further advantages such as the presence of accumulated know-how and materials (neighboring compounds and the like).

### [CITATION LIST]

### [NON PATENT LITERATURE]

[NPL 1] Contractor A. et al., Neuron., 87(4): 699-715 (2015)
[NPL 2] Darnell J. C. et al., Cell., 146(2): 247-261 (2011)
[NPL 3] Liu X. S. et al., Cell., 172(5): 979-992 (2018)

### SUMMARY

### [TECHNICAL PROBLEM]

In light of the foregoing, it is an object of the present invention to provide an agent having therapeutic or prophylactic activity for FXS and to accelerate the development of a therapeutic or prophylactic medicament for FXS as a practical pharmaceutical, through drug repositioning.

### [SOLUTION TO PROBLEM]

The inventors of the present invention induced differentiation of neurons from iPS cells established from FXS patients, and using the survival of these neurons as an index, they performed screening of known compound libraries including drugs already put on the market as pharmaceuticals to search for compounds that increase the expression level of the FMR1 gene. As a result of such screening, the inventors successfully identified the compounds that increase the expression level of the FMR1 gene. Also, the inventors of the present invention found out that administration of such compounds to cells increases the expression level of the FMR1 gene in the cells in a concentration dependent manner. The inventors conducted further research based on this finding, which led to completion of the present invention.

Specifically, the present invention provides the following.
[1] A therapeutic or prophylactic medicament for fragile X syndrome, comprising:
   a compound represented by the following formula (I) or (II), or a salt thereof: 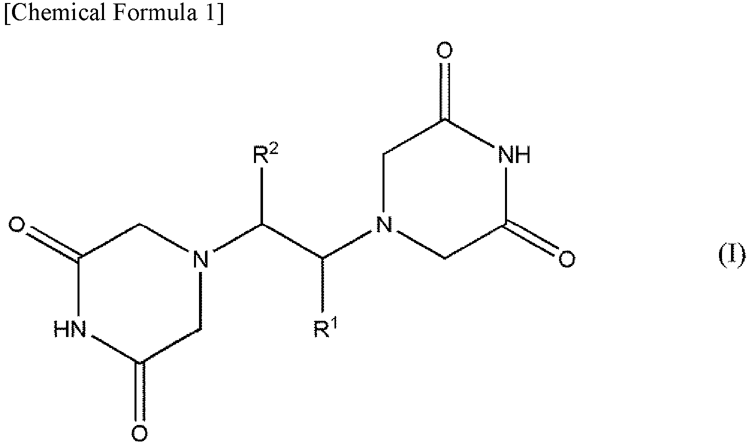 [in the formula (I),
   R¹ and R² each independently represent a hydrogen atom or a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur; or R¹ and R² together may form a C₂-C₆ bridging group], 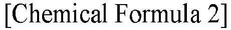 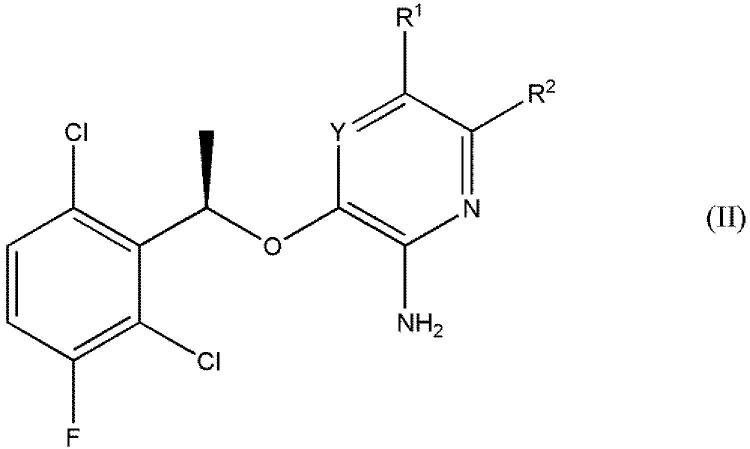 [in the formula (II),
   Y represents a nitrogen atom or CZ [where Z is a hydrogen atom, a halogen atom, CN, or a C₁-C₃ aliphatic group in which each hydrogen atom may be substituted]; and
   R¹ and R² each independently represent a hydrogen atom or a group that is selected from the group consisting of C₁-C₆ aliphatic groups, C₃-C₇ alicyclic hydrocarbon groups, and 5- to 10-membered monocyclic or bicyclic aromatic groups, wherein each hydrogen atom in the group may be substituted and the group may have 1 to 4 heteroatoms each independently selected from nitrogen, oxygen, and sulfur].
[2] The therapeutic or prophylactic medicament according to [1], wherein the formula (I) is represented by the following formula (I-1). 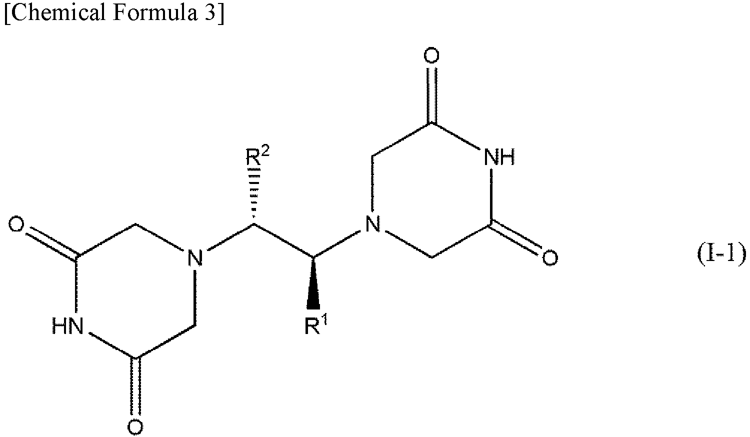 [in the formula (I-1),
   R¹ and R² each independently represent a hydrogen atom or a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur; or R¹ and R² together may form a C₂-C₆ bridging group].
[3-1] The therapeutic or prophylactic medicament according to [1] or [2], wherein R¹ and R² in the formula (I) or (I-1) are each independently a hydrogen atom or a C₁-C₆ aliphatic group in which each hydrogen atom may be substituted.
[3-2] The therapeutic or prophylactic medicament according to any one of [1] to [3-1], wherein R¹ and R² in the formula (I) or (I-1) are each independently a hydrogen atom or a C₁-C₃ aliphatic group in which each hydrogen atom may be substituted.
[3-3] The therapeutic or prophylactic medicament according to any one of [1] to [3-2], wherein R¹ in the formula (I) or (I-1) is a C₁-C₃ aliphatic group.
[3-4] The therapeutic or prophylactic medicament according to any one of [1] to [3-3], wherein R² in the formula (I) or (I-1) is a hydrogen atom.
[4] The therapeutic or prophylactic medicament according to any one of [1] to [3-4], wherein in the formula (I) or (I-1), R¹ is a methyl group and R² is a hydrogen atom.
[5-1] The therapeutic or prophylactic medicament according to [1], wherein Y in the formula (II) is CZ [where Z represents a hydrogen atom, a halogen atom, or CN].
[5-2] The therapeutic or prophylactic medicament according to [1] or [5-1], wherein Y in the formula (II) is CH.
[6-1] The therapeutic or prophylactic medicament according to any one of [1], [5-1], and [5-2], wherein R¹ in the formula (II) is a 5- to 10-membered monocyclic or bicyclic aromatic group in which each hydrogen atom may be substituted and that may have 1 to 4 heteroatoms each independently selected from nitrogen, oxygen, and sulfur.
[6-2] The therapeutic or prophylactic medicament according to any one of [1] and [5-1] to [6-1], wherein R¹ in the formula (II) is a 5- to 10-membered monocyclic aromatic group in which each hydrogen atom may be substituted and that may have 1 to 4 heteroatoms each independently selected from nitrogen, oxygen, and sulfur.
[6-3] The therapeutic or prophylactic medicament according to any one of [5-1] to [6-2], wherein substitution of a hydrogen atom is substitution of the hydrogen atom with a C₃-C₇ alicyclic hydrocarbon group that may have 1 to 4 heteroatoms each independently selected from nitrogen, oxygen, and sulfur.
[6-4] The therapeutic or prophylactic medicament according to any one of [6-1] to [6-3], wherein at least one of the heteroatoms is a nitrogen atom.
[6-5] The therapeutic or prophylactic medicament according to any one of [6-1] to [6-4], wherein all the heteroatoms are nitrogen atoms.
[7] The therapeutic or prophylactic medicament according to any one of [1] and [5-1] to [6-5], wherein the formula (II) is the following formula (II-1). 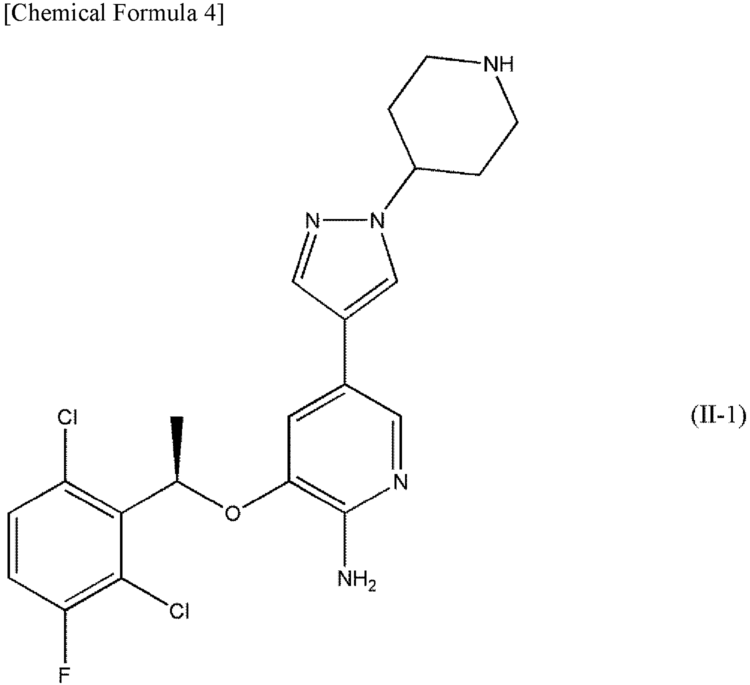
[8] An agent for promoting FMR1 gene expression, comprising:
   a compound represented by the following formula (I) or (II), or a salt thereof: 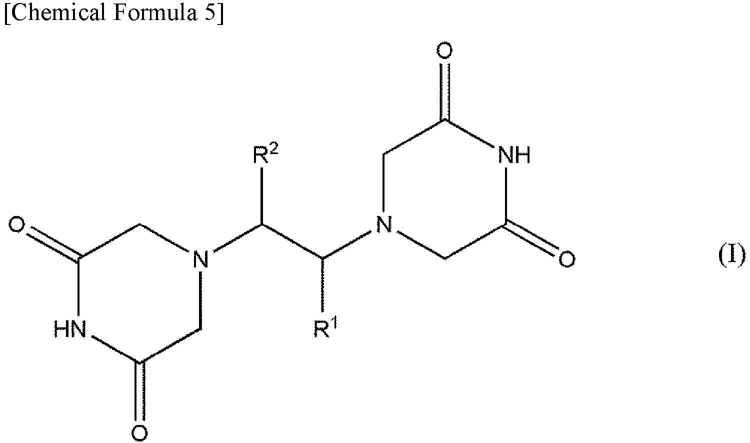 [in the formula (I),
   R¹ and R² each independently represent a hydrogen atom or a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur; or R¹ and R² together may form a C₂-C₆ bridging group], 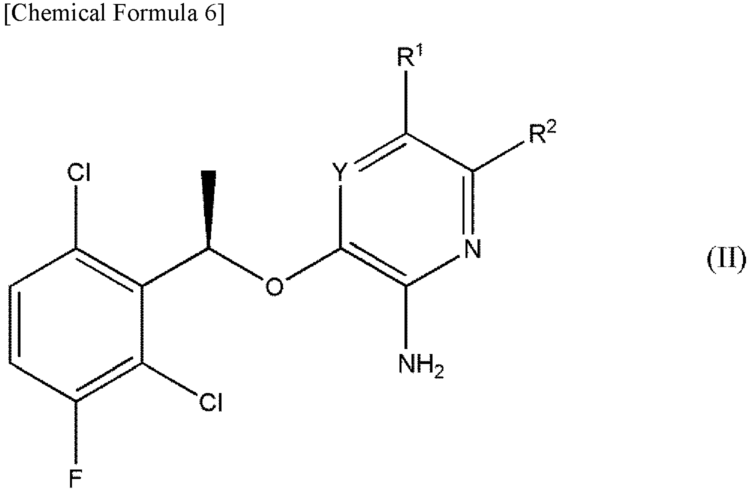 [in the formula (II),
   Y represents a nitrogen atom or CZ [where Z is a hydrogen atom, a halogen atom, CN, or a C₁-C₃ aliphatic group in which each hydrogen atom may be substituted]; and
   R¹ and R² each independently represent a hydrogen atom or a group that is selected from the group consisting of C₁-C₆ aliphatic groups, C₃-C₇ alicyclic hydrocarbon groups, and 5- to 10-membered monocyclic or bicyclic aromatic groups, wherein each hydrogen atom in the group may be substituted and the group may have 1 to 4 heteroatoms each independently selected from nitrogen, oxygen, and sulfur].
[9] A method for treating or preventing fragile X syndrome in a mammal, comprising:
   administering to the mammal an effective amount of a compound represented by the following formula (I) or (II), or a salt thereof: 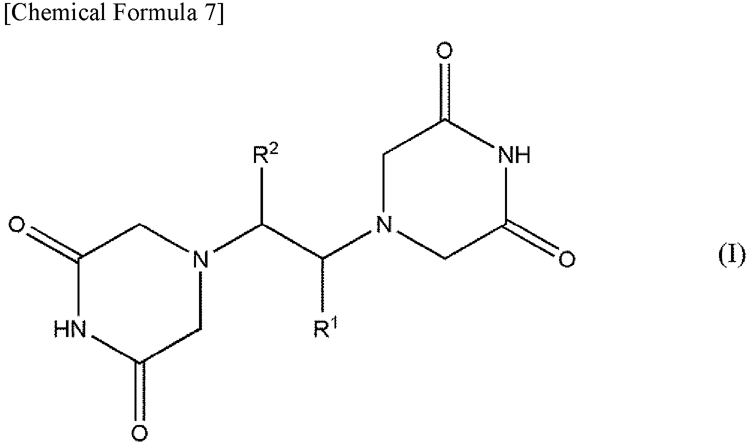 [in the formula (I),
   R¹ and R² each independently represent a hydrogen atom or a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur; or R¹ and R² together may form a C₂-C₆ bridging group], 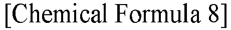 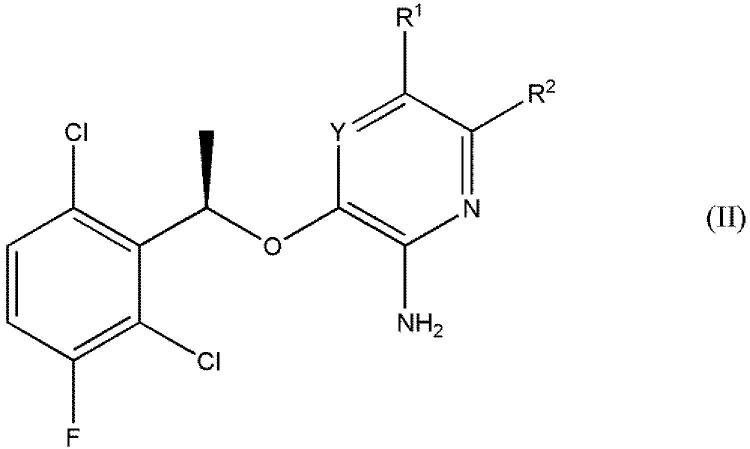 [in the formula (II),
   Y represents a nitrogen atom or CZ [where Z is a hydrogen atom, a halogen atom, CN, or a C₁-C₃ aliphatic group in which each hydrogen atom may be substituted]; and
   R¹ and R² each independently represent a hydrogen atom or a group that is selected from the group consisting of C₁-C₆ aliphatic groups, C₃-C₇ alicyclic hydrocarbon groups, and 5- to 10-membered monocyclic or bicyclic aromatic groups, wherein each hydrogen atom in the group may be substituted and the group may have 1 to 4 heteroatoms each independently selected from nitrogen, oxygen, and sulfur].
[10] A compound represented by the following formula (I) or (II), or a salt thereof for use in treatment or prevention of fragile X syndrome: 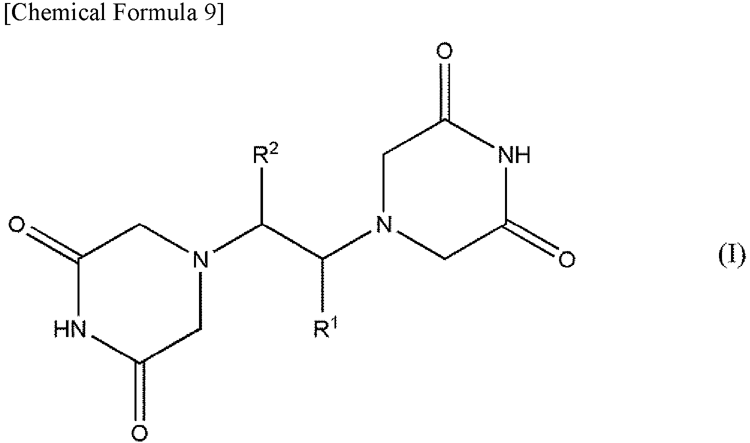 [in the formula (I),
   R¹ and R² each independently represent a hydrogen atom or a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur; or R¹ and R² together may form a C₂-C₆ bridging group], 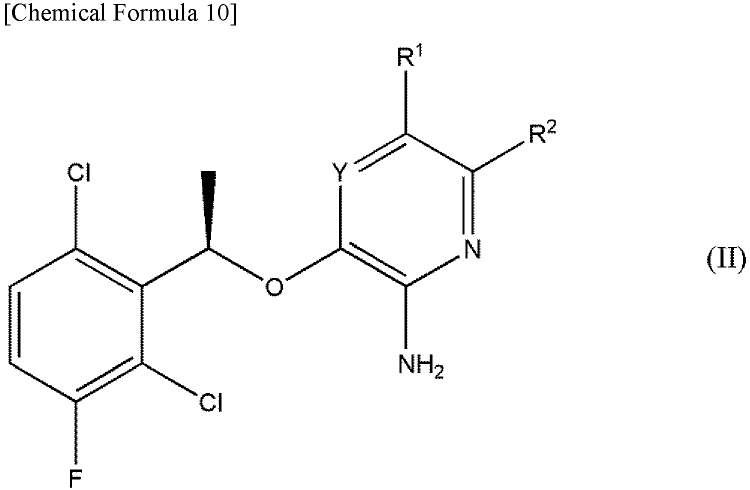 [in the formula (II),
   Y represents a nitrogen atom or CZ [where Z is a hydrogen atom, a halogen atom, CN, or a C₁-C₃ aliphatic group in which each hydrogen atom may be substituted]; and
   R¹ and R² each independently represent a hydrogen atom or a group that is selected from the group consisting of C₁-C₆ aliphatic groups, C₃-C₇ alicyclic hydrocarbon groups, and 5- to 10-membered monocyclic or bicyclic aromatic groups, wherein each hydrogen atom in the group may be substituted and the group may have 1 to 4 heteroatoms each independently selected from nitrogen, oxygen, and sulfur].
[11] Use of a compound represented by the following formula (I) or (II), or a salt thereof for manufacture of a therapeutic or prophylactic medicament for fragile X syndrome: 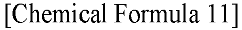 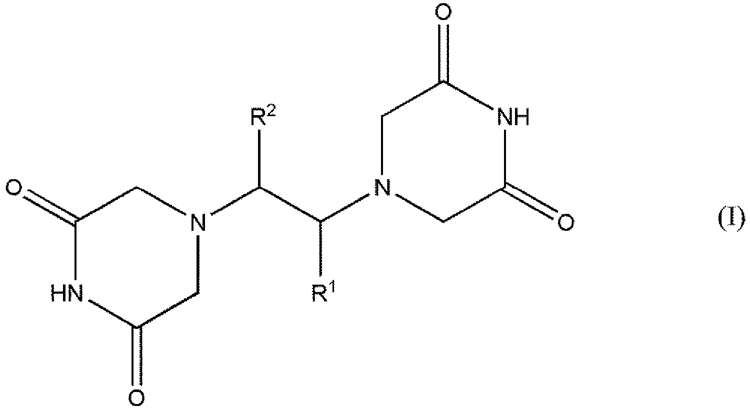 [in the formula (I),
   R¹ and R² each independently represent a hydrogen atom or a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur; or R¹ and R² together may form a C₂-C₆ bridging group], 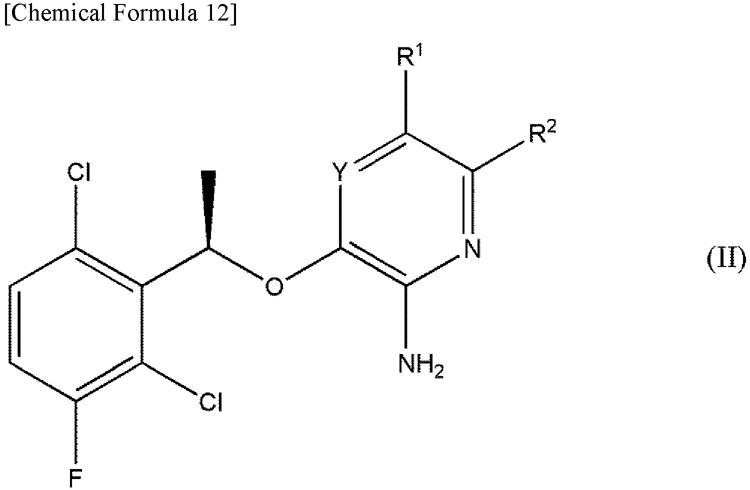 [in the formula (II),
   Y represents a nitrogen atom or CZ [where Z is a hydrogen atom, a halogen atom, CN, or a C₁-C₃ aliphatic group in which each hydrogen atom may be substituted]; and
   R¹ and R² each independently represent a hydrogen atom or a group that is selected from the group consisting of C₁-C₆ aliphatic groups, C₃-C₇ alicyclic hydrocarbon groups, and 5- to 10-membered monocyclic or bicyclic aromatic groups, wherein each hydrogen atom in the group may be substituted and the group may have 1 to 4 heteroatoms each independently selected from nitrogen, oxygen, and sulfur].

### [ADVANTAGEOUS EFFECTS OF INVENTION]

The present invention enables treatment or prevention of FXS. Notably, since the present invention uses an existing drug with confirmed safety as an active ingredient, it is expected that the present invention can shorten the development period required until clinical application.

### BRIEF DESCRIPTION OF DRAWINGS

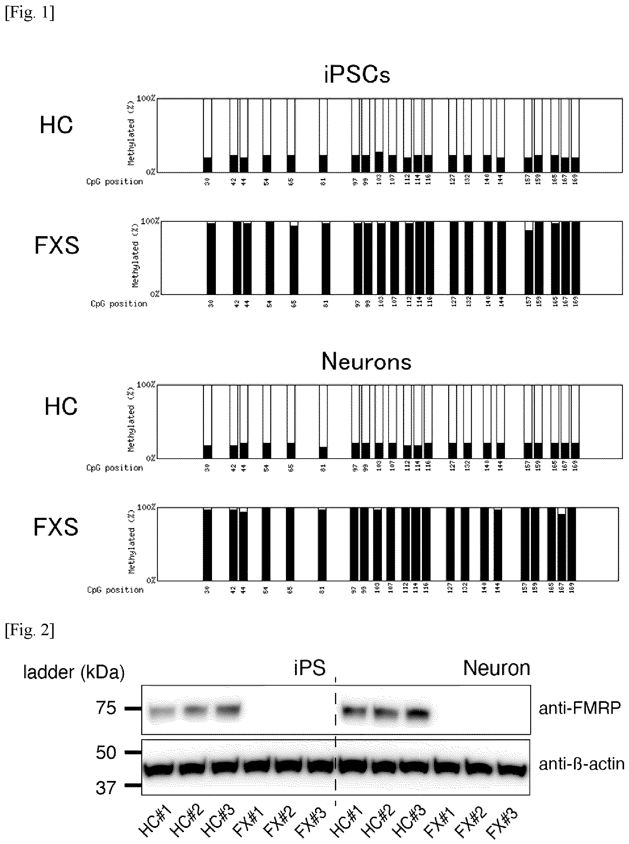

FIG. 1 shows the results of measuring methylation of the promoter region in the FMR1 gene in: iPS cells derived from a healthy control (HC) and an FXS patient; and neurons generated from these cells. The promoter region of the FMR1 gene is highly methylated in the iPS cells of the FXS patients and the neurons generated therefrom. In FIG. 1, CpG positions are, from left to right, 30, 42, 44, 54, 65, 81, 97, 99, 103, 107, 112, 114, 116, 127, 132, 140, 144, 157, 159, 165, 167, and 169.
FIG. 2 shows the results of measuring the expression of FMRP in: iPS cells derived from healthy controls (HC) and FXS patients; and neurons generated from these cells. The expression of FMRP is not observed in the iPS cells of the FXS patients and the neurons generated therefrom.

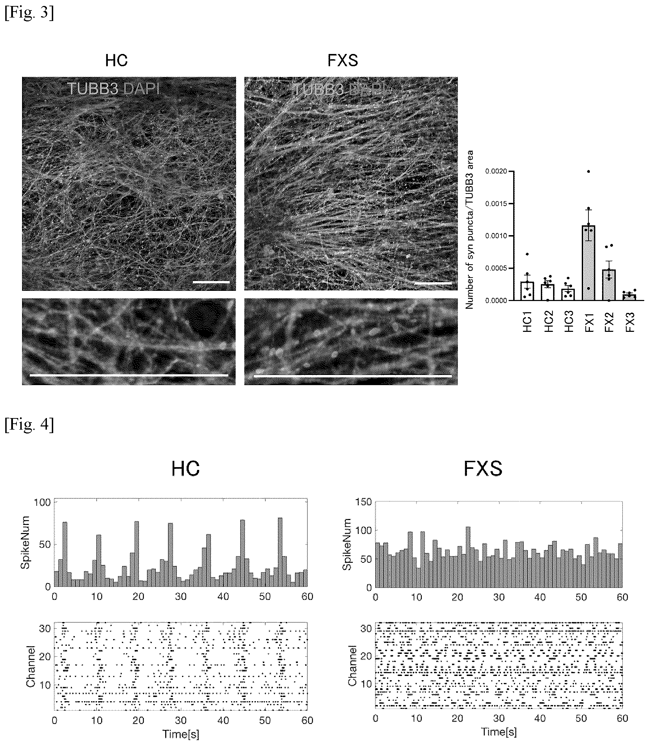

FIG. 3 shows the results of measuring the number of synaptic puncta and the neuronal activity in neurons generated from iPS cells derived from the healthy controls (HC) and the FXS patients. The neurons generated from the iPS cells of the FXS patients show an increase in the number of synapses stained with Synapsin-1 antibody. Error bar: standard error.
FIG. 4 shows the results of detecting the neuronal activity of neurons derived from iPS cells of the healthy controls (HC) and the FXS patients in the form of raster plots. In the neurons generated from the iPS cells of the FXS patients, enhanced neuronal activity is observed in evaluation using a microelectrode array (MEA).

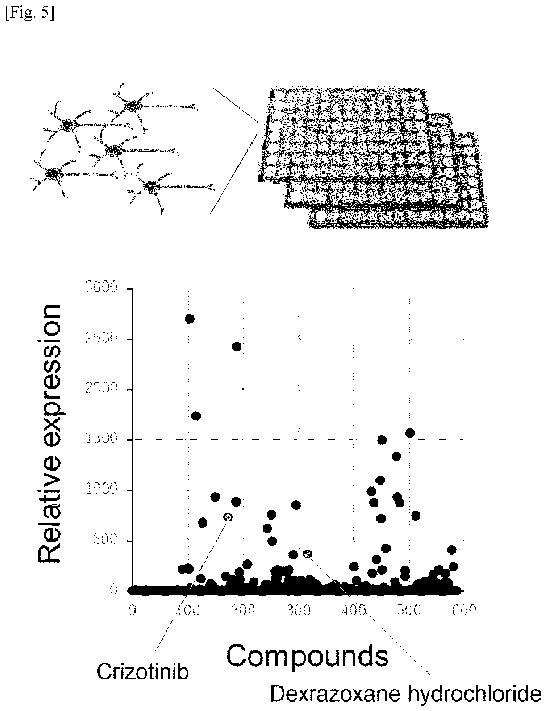

FIG. 5 illustrates the overview of screening of about 600 compounds to search for compounds that increase the expression level of FMR1, and also shows the screening result.

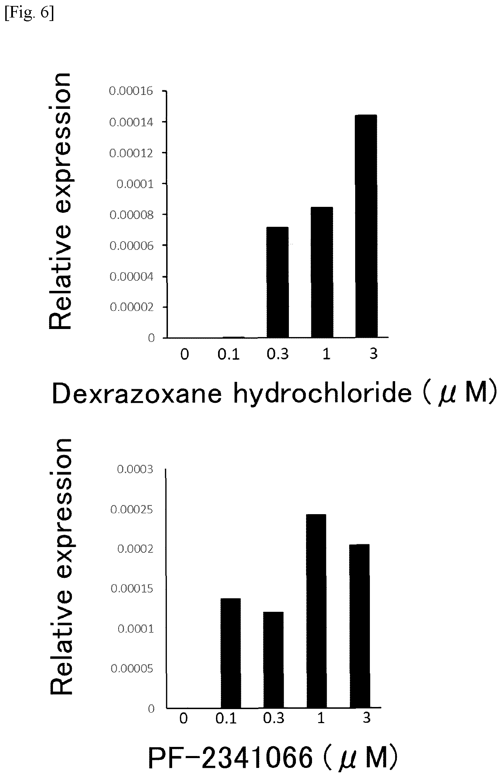

FIG. 6 indicates that two compounds extracted from the hit compounds increased the expression of FMRP.

### DESCRIPTION OF EMBODIMENTS

As shown in examples to be described below, the inventors of the present invention found out that administration of dexrazoxane hydrochloride or crizotinib to cells increases the expression level of the FMR1 gene in the cells. Since the symptoms of fragile X syndrome (FXS) can be alleviated by increasing the expression level of the FMR1 gene in vivo, dexrazoxane or an analog thereof and crizotinib or an analog thereof can be used for the treatment or prevention of FXS. Thus, the present invention provides a therapeutic or prophylactic medicament for FXS (hereinafter also referred to as "pharmaceutical of the present invention"), comprising dexrazoxane or an analog thereof or crizotinib or an analog thereof (hereinafter also referred to as "compound of the present invention"). Unless otherwise stated, a therapeutic or prophylactic medicament (or method) for FXS encompasses a pharmaceutical (or method) capable of treating and preventing the disease.

The term "therapeutic medicament" as used herein shall be taken to encompass not only pharmaceuticals aimed at definitive therapy for FXS but also, for example, pharmaceuticals aimed at inhibiting the progression of FXS, pharmaceuticals aimed at alleviating the symptoms (for example, to the extent to achieve a state of minimal manifestation (MM) where the symptoms do not bother one's life or work activities), and pharmaceuticals for alleviating sequelae. For example, since FXS progresses over a long period of time (usually over one year), the progression of symptoms can be prevented by starting the treatment at an early stage. The term "prophylactic medicament" as used herein shall be taken to encompass not only pharmaceuticals aimed at reducing the risk of developing FXS in a subject who has not developed FXS but also pharmaceuticals aimed at reducing the risk of recurrence of FXS in a subject who has developed FXS. For example, it is also possible to prevent the development of FXS in a patient who potentially has a genetic background susceptible to FXS by administering the pharmaceutical of the present invention before the patient shows the symptoms of FXS. The same applies to the terms "method for treating" and "method for preventing".

As the pharmaceutical of the present invention, the compound of the present invention, which is an active ingredient, may be administered alone orally or parenterally, or alternatively, the compound of the present invention may be mixed with a pharmacologically acceptable carrier, excipient, diluent, and the like to form a pharmaceutical composition in a suitable dosage form and the thus-obtained pharmaceutical composition may be administered orally or parenterally. The pharmaceutical of the present invention can be administered to mammals (e.g., humans, rats, mice, guinea pigs, rabbits, sheep, horses, pigs, cows, dogs, cats, and monkeys). Thus, the present invention also provides a method for treating or preventing FXS in a mammal, comprising administering an effective amount of the compound of the present invention to the mammal.

As used herein, FXS refers to a disease caused by expansion of CGG codon repeats (also referred to as "CGG repeats") in the 5' untranslated region in the first exon of the FMR1 gene, and typical FXS patients have more than 200 CGG repeats in the FRM1 gene. Suppression of the FMR1 gene expression is caused by hypermethylation of such repeats and the neighboring CpG island, and this is considered as the core of the pathophysiology of FXS.

In addition, the compound of the present invention can also be used as an agent for promoting FMR1 gene expression. Thus, in another aspect, the present invention provides an agent for promoting FMR1 gene expression comprising the compound of the present invention (hereinafter also referred to as "agent for promoting expression of the present invention"). As used herein, the agent for promoting expression of the present invention encompasses any agents as long as they promote the expression of the FMR1 gene at the cellular level or at the biological level. The term "gene expression" as used herein is intended to encompass at least "production of a functional protein encoded by mRNA of FMR1", but also further encompasses "production of mRNA of FMR1", unless otherwise stated. Accordingly, promotion of the expression of the FMR1 gene may encompass not only an increase in the abundance of a functional protein encoded by the gene present in cells but also an increase in the abundance of mRNA transcribed from the gene in the cells, as a result of administering the compound of the present invention.

The agent for promoting expression of the present invention is prepared in the form of a common pharmaceutical composition or pharmaceutical formulation or in the form of a cosmetic product or food, and administered orally or parenterally. The agent for promoting expression of the present invention can also be used as a reagent. The agent for promoting expression of the present invention can be administered to, for example, subjects including humans (e.g., mammals and cells, tissues, and organs of mammals). Thus, the present invention also provides a method for promoting the expression of the FMR1 gene in a subject, comprising administering the compound of the present invention to the subject.

Dexrazoxane or an analog thereof used in the present invention may be, for example, a compound represented by the following formula (I) or a salt thereof.

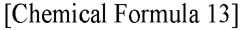

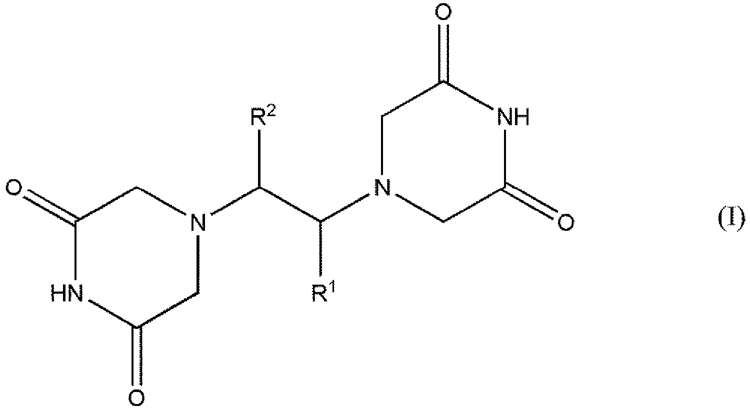

[In the formula (I),
R¹ and R² each independently represent a hydrogen atom or a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur; or R¹ and R² together may form a C₂-C₆ bridging group].

In one embodiment, the compound represented by the formula (I) or a salt thereof may be, for example, a compound represented by the following formula (I-1) or a salt thereof.

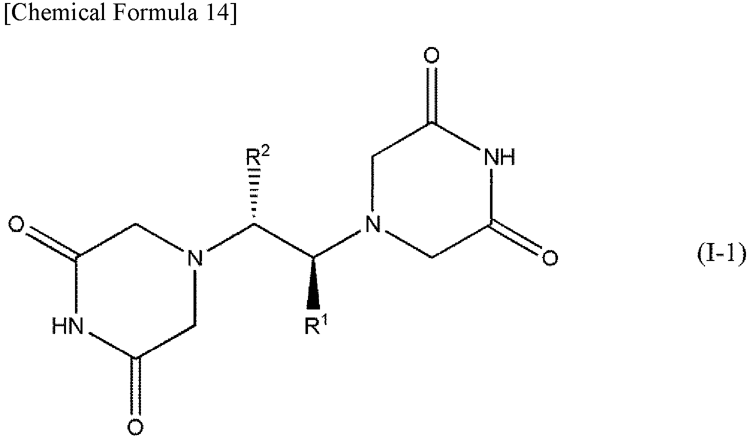

[In the formula (I-1), the definitions of R¹ and R² are the same as those in the formula (I)].

In the compound represented by the above formula (I) or (I-1) or a salt thereof, it is preferable that R¹ and R² are each independently a hydrogen atom or a C₁-C₆ aliphatic group in which each hydrogen atom may be substituted. In particular, it is more preferable that at least one of R¹ and R² (preferably R¹) is a C₁-C₃ (preferably C₁) aliphatic group (preferably alkyl group) in which each hydrogen atom may be substituted (preferably not be substituted). It is also preferable that R² is a hydrogen atom. Thus, in a preferable embodiment, dexrazoxane or an analog thereof may be the compound represented by the above formula (I) or (I-1), where R¹ is a C₁-C₃ (preferably C₁) aliphatic group (preferably alkyl group) and R² is a hydrogen atom, or a salt thereof.

Specific examples of dexrazoxane or an analog thereof include a compound represented by the following formula (I-1-a) (i.e., dexrazoxane; 4-[(2S)-2-(3,5-dioxopiperazin-1-yl)propyl] piperazine-2,6-dione) (this is a compound represented by the above formula (I-1), where R¹ is a methyl group and R² is a hydrogen atom). Specific examples of a salt of the above compound include a salt represented by the following formula (I-1-b) (i.e., dexrazoxane hydrochloride; 4-[(2S)-2-(3,5-dioxopiperazin-1-yl)propyl]piperazine-2,6-dione;hydrochloride).

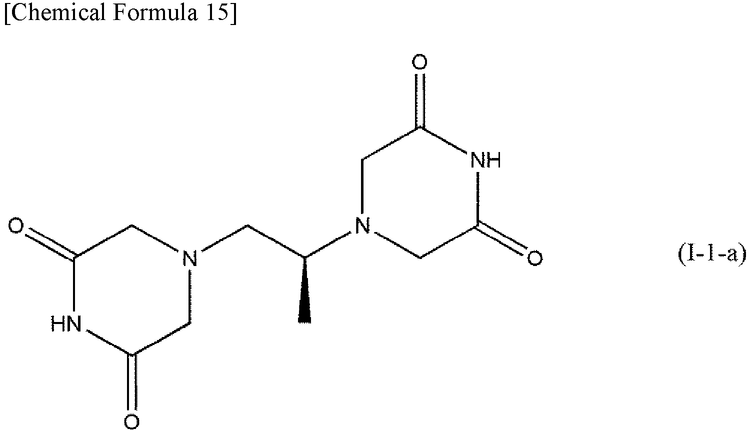

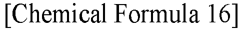

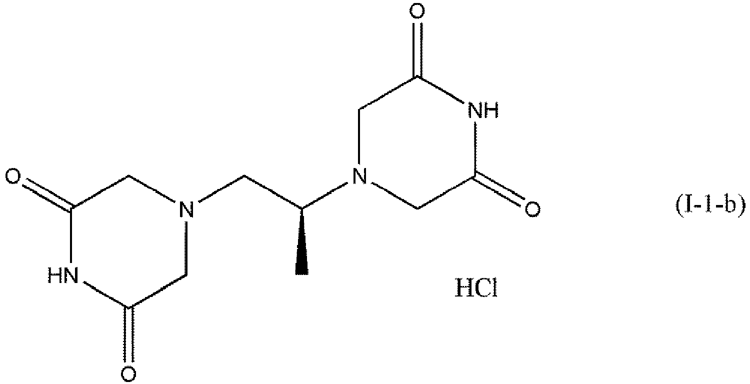

Crizotinib or an analog thereof used in the present invention may be, for example, a compound represented by the following formula (II) or a salt thereof.

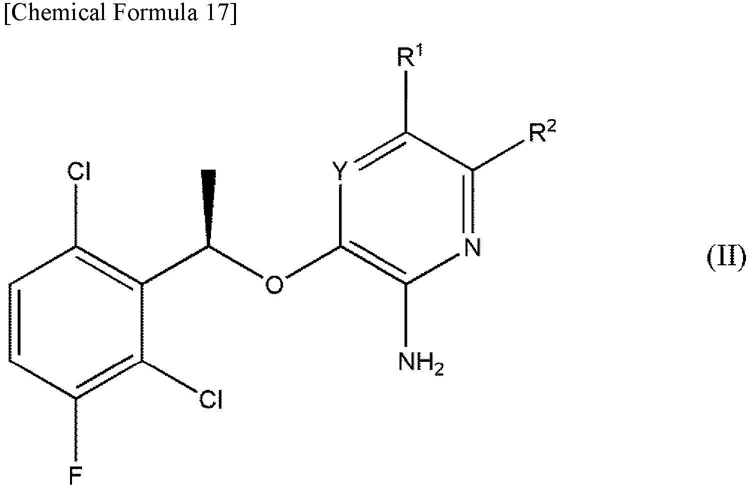

[In the formula (II),
Y represents a nitrogen atom or CZ [where Z is a hydrogen atom, a halogen atom, CN, or a C₁-C₃ aliphatic group in which each hydrogen atom may be substituted]; and
R¹ and R² each independently represent a hydrogen atom or a group that is selected from the group consisting of C₁-C₆ aliphatic groups, C₃-C₇ alicyclic hydrocarbon groups, and 5- to 10-membered monocyclic or bicyclic aromatic groups, wherein each hydrogen atom in the group may be substituted and the group may have 1 to 4 heteroatoms each independently selected from nitrogen, oxygen, and sulfur].

The above formula (II) can also be represented as the following formula (II-a).

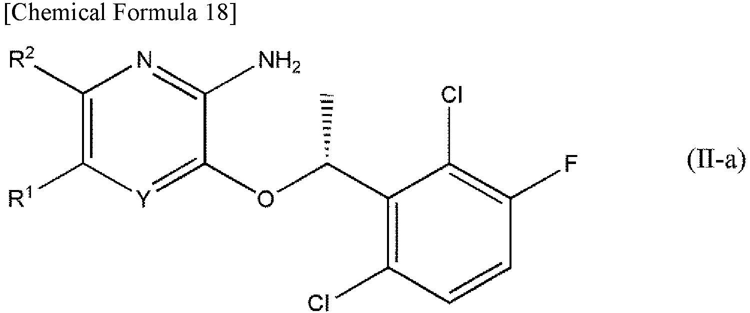

[In the formula (II-a), the definitions of R¹, R², and Y are the same as those in the formula (II)].

In one embodiment, crizotinib or an analog thereof may be, for example, a compound represented by the formula (II), where Y is CZ [where Z is a hydrogen atom, a halogen atom, or CN], or a salt thereof. In particular, preferred is the above compound in which Z is a hydrogen atom, or a salt thereof.

In another embodiment, crizotinib or an analog thereof may be a compound represented by the formula (II), where R¹ is a 5- to 10-membered (preferably 5-membered) monocyclic or bicyclic aromatic group (preferably monocyclic aromatic group) in which each hydrogen atom may be substituted and that may have 1 to 4 (preferably 2) heteroatoms each independently selected from nitrogen, oxygen, and sulfur, or a salt thereof. Also, it is preferable that substitution of a hydrogen atom is substitution of the hydrogen atom with a C₃-C₇ (preferably C₆) alicyclic hydrocarbon group (preferably cycloalkyl group) that may have 1 to 4 (preferably 1) heteroatoms each independently selected from nitrogen, oxygen, and sulfur (preferably 1 to 4 heteroatoms are nitrogen atoms). When R¹ has heteroatoms, it is preferable that at least one (preferably all) of the heteroatoms is a nitrogen atom.

Specific examples of crizotinib or an analogue thereof include a compound represented by the following formula (II-1) (i.e., crizotinib; 3-[(1R)-1-(2,6-dichloro-3-fluorophenyl)ethoxy]-5-(1-piperidin-4-ylpyrazol-4-yl)pyridin-2-amine). The above formula (II-1) can also be represented as the following formula (II-1-a).

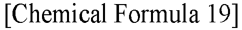

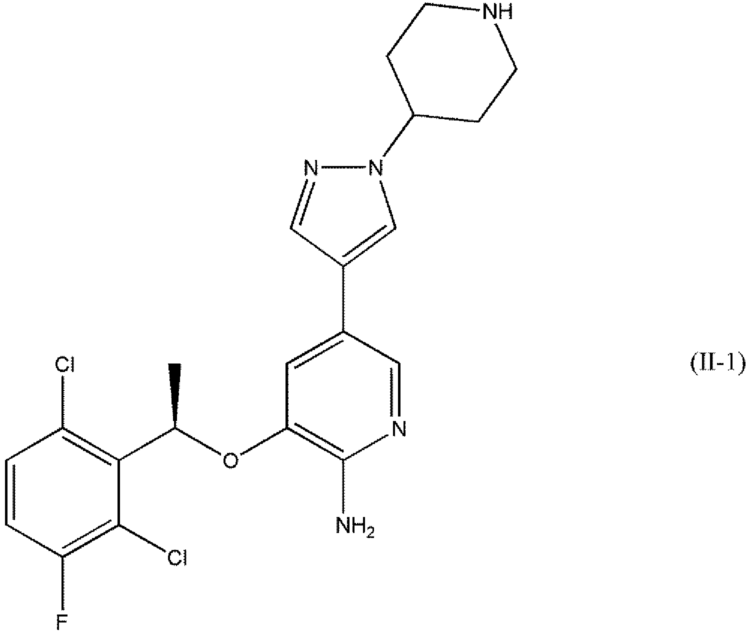

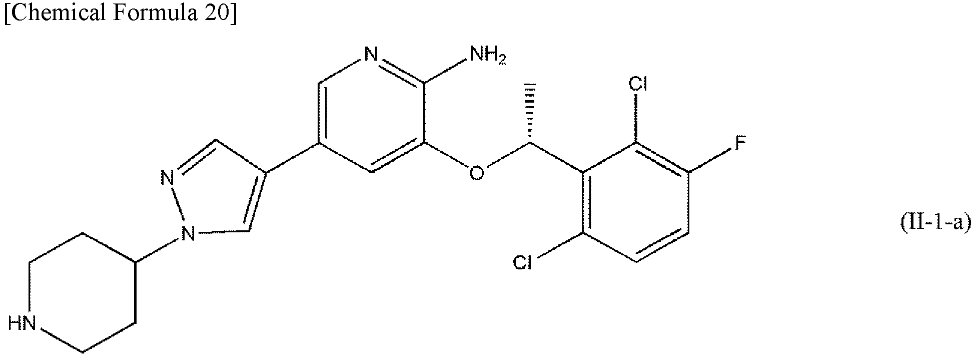

As used herein, the term "aliphatic group" means a straight or branched hydrocarbon chain that is fully saturated or contains one or more unsaturated bonds. Examples of the aliphatic group include straight or branched alkyl groups (e.g., methyl group, ethyl group, propyl group, isopropyl group, butyl group, 2-butyl group, 2-methylpropyl group, 1,1-dimethylethyl group, pentyl group, 3-pentyl group, 3-methylbutyl group, hexyl group, and 3-hexyl group), alkenyl groups (e.g., vinyl group, allyl group, 2-propynyl group, 2-butenyl group 3-methyl-2-butenyl group, and 3-hexenyl group), alkynyl groups (e.g., ethynyl group, 1-propynyl group, 2-propynyl group, 1-butynyl group, 2-butynyl group, 3-butynyl group, 1-pentynyl group, 2-pentynyl group, 3-pentynyl group, 4-pentynyl group, 1-hexynyl group, 2-hexynyl group, 3-hexynyl group, 4-hexynyl group, 5-hexynyl group, and 4-methyl-2-pentynyl group). The term "alicyclic hydrocarbon group" means a monocyclic or bicyclic hydrocarbon group that is fully saturated or contains one or more unsaturated bonds and that does not belong to aromatic hydrocarbon groups, or means an aliphatic group having such a hydrocarbon group. Examples of the alicyclic hydrocarbon group include cycloalkyl groups (e.g., cyclopropyl group, cyclobutyl group, cyclopentyl group, cyclohexyl group, and cycloheptyl group), cycloalkenyl groups (e.g., cyclopropenyl group, cyclobutenyl group, cyclopentenyl group, cyclohexenyl group, and cycloheptenyl group), (cycloalkyl)alkyl groups, (cycloalkenyl)alkyl groups, and (cycloalkyl)alkenyl groups.

The monocyclic or bicyclic aromatic group may be, for example, an aryl group or a heteroaryl group. Examples of the aryl group include aryl groups having 10 or less carbon atoms, such as a phenyl group and a naphthyl group. Examples of the heteroaryl group include 5- to 6-membered monocyclic groups containing 1 to 2 nitrogen atoms, 5- or 6-membered monocyclic groups containing 1 to 2 nitrogen atoms and one oxygen or sulfur atom, 5-membered monocyclic groups containing one oxygen or sulfur atom, and bicyclic groups that contain 1 to 4 nitrogen atoms and in which a 6-membered ring is fused with a 5- or 6-membered ring, and specific examples thereof include a 2-pyridyl group, 3-pyridyl group, 4-pyridyl group, 2-thienyl group, 3-thienyl group, 3-oxadiazolyl group, 1-imidazolyl group, 2-imidazolyl group, 2-thiazolyl group, 3-isothiazolyl group, 2-oxazolyl group, 3-isoxazolyl group, 2-furyl group, 3-furyl group, 3-pyrrolyl group, 8-quinolyl group, 2-quinazolinyl group, and 8-purinyl group.

Examples of the halogen atom include fluorine, chlorine, bromine, and iodine.

As used herein, "each hydrogen atom may be substituted (or is substituted)" means that at least one of hydrogen atoms present in a group may be substituted (or is substituted) with another atom or group. That is, a group in which each hydrogen atom may be substituted (or is substituted) is, in other words, a group that may have (or has) a substituent. In the present specification, the substituent may be, for example, a halogen atom, cyano group, benzyloxy group, trifluoromethyl group, hydroxy group, lower alkoxy group, lower alkanoyloxy group, amino group, mono-lower alkylamino group, di-lower alkylamino group, carbamoyl group, lower alkylaminocarbonyl group, di-lower alkylaminocarbonyl group, lower alkoxycarbonylamino group, carboxyl group, lower alkoxycarbonyl group, lower alkylthio group, lower alkylsulfinyl group, lower alkylsulfonyl group, lower alkanoylamino group, lower alkylsulfonamide group, phthalimido group, heteroaryl group, aryl with a substituent(s) (substituted aryl), heteroaryl with a substituent(s) (substituted heteroaryl), saturated heterocyclic group, or group represented by the formula: -NR⁷R⁸ (where R⁷ and R⁸ each independently represent a hydrogen atom, lower alkoxy group, lower alkyl group, substituted lower alkyl group, cycloalkyl group, or aralkyl group, or R⁷ and R⁸ are bonded together and they represent, together with nitrogen atoms to which they are bound, a saturated cyclic amino group having 4 to 8 carbon atoms in its ring). Examples of the heteroaryl group are the same as those given above. Examples of the saturated heterocyclic group include 5- to 8-membered groups having one nitrogen atom, such as 1-piperidinyl and 1-pyrrolidinyl, 6- to 8-membered groups having two nitrogen atoms, and 6- to 8-membered groups having one nitrogen atom and one oxygen atom. The substituted alkyl group may be a C₁-C₆ alkyl group substituted with a cycloalkyl group or with a substituted cycloalkyl group, or an aralkyl group or substituted aralkyl group. Examples of the aralkyl group and substituted aralkyl group include C₁-C₆ alkyl groups substituted with the above-described aryl groups or a substituted aryl group, such as, for example, a benzyl group, 1-phenylethyl group, 2-phenylethyl group, and 2-naphthylmethyl group. In the present specification, an unsubstituted group is intended in the absence of the description "each hydrogen atom may be substituted", unless otherwise stated. In the present specification, the term "lower" means having 5 or less (preferably 3 or less) carbon atoms.

As dexrazoxane or an analog thereof or crizotinib or an analog thereof, commercially available products may be used, or each of these compounds can be produced by a method known per se.

Dexrazoxane or an analog thereof can be produced according to the methods described in, for example, U.S. Patent No. 3,941,790, International Patent Publication No. WO01/19358, and literatures cited therein. Dexrazoxane is commercially available from Kissei Pharmaceutical Co., Ltd. and other companies as a drug for inhibiting histological damage caused by extravasation (EV) of anthracycline anticancer agents.

Crizotinib or an analog thereof can be produced according to the methods described in, for example, U.S. Patent No. 7,858,643 and literatures cited therein. Crizotinib is commercially available from Pfizer Inc. as an antineoplastic drug (trade name: XALKORI (register trade mark).

Dexrazoxane or an analog thereof or crizotinib or an analog thereof shall be taken to encompass not only the free form thereof but also pharmacologically acceptable salts thereof. Examples of the pharmacologically acceptable salts, which vary depending on the types of compounds, include: base addition salts including inorganic base salts such as alkali metal salts (such as sodium salts and potassium salts), alkaline-earth metal salts (such as calcium salts and magnesium salts), aluminum salts, and ammonium salts and organic base salts such as trimethylamine, triethylamine, pyridine, picoline, ethanolamine, diethanolamine, triethanolamine, dicyclohexylamine, and N,N'-dibenzylethylenediamine; and acid addition salts including inorganic acid salts such as hydrochloride, hydrobromide, sulfate, hydroiodide, nitrate, and phosphate and organic acid salts such as citrate, oxalate, acetate, formate, propionate, benzoate, trifluoroacetate, maleate, tartrate, methanesulfonate, benzenesulfonate, and paratoluenesulfonate.

In the case where dexrazoxane or an analog thereof or crizotinib or an analog thereof has isomers such as optical isomers, stereoisomers, regioisomers, and rotamers, any of the isomers and mixtures thereof are also encompassed in the compound of the present invention. For example, in the case where dexrazoxane or an analog thereof or crizotinib or an analog thereof has optical isomers, an optical isomer separated from a racemate is also encompassed in the compound of the present invention. Each of these isomers can be obtained as a single product by a method known per se, such as a synthesis method, a separation method (e.g., concentration, solvent extraction, column chromatography, or recrystallization), or an optical resolution method (e.g., fractional crystallization, a chiral column method, or a diastereomer method).

Dexrazoxane or an analog thereof or crizotinib or an analog thereof may be in the form of crystals, and regardless of whether it has only one crystal form or a mixture of crystal forms, it is encompassed in the compound of the present invention. Crystals can be produced by causing crystallization using a crystallization method known per se.

Dexrazoxane or an analog thereof or crizotinib or an analog thereof may be a solvate (e.g., a hydrate) or an ansolvate (e.g., a non-hydrate), and both of them are encompassed in the compound of the present invention.

Compounds labeled with an isotope (e.g., ³H, ¹⁴C, ³⁵S, or ¹²⁵I) or the like are also encompassed in the compound of the present invention.

The composition for oral administration may be in a solid or liquid dosage form, and specific examples of the dosage form include tablets (including sugar-coated tablets and filmcoated tablets), pills, granules, powdered drugs, capsules (including soft capsules), syrups, emulsions, and suspensions. On the other hand, the composition for parenteral administration may be, for example, an injection or a suppository, and the injection may encompass dosage forms such as an intravenous injection, subcutaneous injection, intradermal injection, intramuscular injection, and intravenous infusion. These formulations are produced by well-known methods using additives, examples of which include excipients (e.g., organic excipients including: sugar derivatives such as lactose, saccharose, glucose, mannitol, and sorbitol; starch derivatives such as corn starch, potato starch, α-starch, and dextrin; cellulose derivatives such as crystalline cellulose; gum arabic; dextran; and pullulan; and inorganic excipients including: silicate derivatives such as light anhydrous silicic acid, synthetic aluminum silicate, calcium silicate, and magnesium aluminometasilicate; phosphates such as calcium hydrogen phosphate; carbonates such as calcium carbonate; and sulfates such as calcium sulfate), lubricants (e.g., stearic acid and metal stearates such as calcium stearate and magnesium stearate; talc; colloidal silica; waxes such as beeswax and spermaceti; boric acid; adipic acid; sulfates such as sodium sulfate; glycol; fumaric acid; sodium benzoate; DL-leucine; lauryl sulfates such as sodium lauryl sulfate and magnesium lauryl sulfate; silicates such as silicic acid anhydrides and silicic acid hydrates; and the above-described starch derivatives), binders (e.g., hydroxypropylcellulose, hydroxypropylmethylcellulose, polyvinylpyrrolidone, macrogol, and the same compounds as those given above as examples of the excipients), disintegrants (e.g., cellulose derivatives such as low-substituted hydroxypropylcellulose, carboxymethylcellulose, carboxymethylcellulose calcium, and internally cross-linked sodium carboxymethylcellulose; and chemically modified starches and celluloses, such as carboxymethyl starch, sodium carboxymethyl starch, and cross-linked polyvinylpyrrolidone), emulsifiers (e.g., colloidal clays such as bentonite and Veegum; metal hydroxides such as magnesium hydroxide and aluminum hydroxide; anionic surfactants such as sodium lauryl sulfate and calcium stearate; cationic surfactants such as benzalkonium chloride; and non-ionic surfactants such as polyoxyethylene alkyl ethers, polyoxyethylene sorbitan fatty acid esters, and sucrose fatty acid esters), stabilizers (para-hydroxybenzoic acid esters such as methylparaben and propylparaben; alcohols such as chlorobutanol, benzyl alcohol, and phenylethyl alcohol; benzalkonium chloride; phenols such as phenol and cresol; thimerosal; dehydroacetic acid; and sorbic acid), taste and odor masking agents (e.g., commonly used sweeteners, acidulants, and flavors), and diluents.

The dose of the compound of the present invention, which is an active ingredient of the pharmaceutical or agent for promoting expression of the present invention, may vary according to various conditions including the type of compound and the symptoms, age, weight, and drug acceptability of a subject to which the compound is administered, and the compound can be administered to an adult one to six times a day with the lower limit per dose being 0.1 mg (suitably 0.5 mg) and the upper limit per dose being 1000 mg (suitably 500 mg) for oral administration and with the lower limit per dose being 0.01 mg (suitably 0.05 mg) and the upper limit per dose being 100 mg (suitably 50 mg) for parenteral administration. The dose may be increased or decreased in consideration of the symptoms. In particular, when compounds according to the present invention are compounds that have already been put on the market as pharmaceuticals for diseases other than the above-described disease, an appropriate dose can be selected for each compound within the range where the safety of the compound is confirmed.

The pharmaceutical or agent for promoting expression of the present invention can also be used in combination with therapeutic or prophylactic medicaments for FXS (including those currently in clinical trials) (e.g., metabotropic glutamate receptor antagonists, GABA receptor modulators, minocycline, selective serotonin reuptake inhibitors, lovastatin, metformin, and cannabidiol). When any of these agents is used as a co-agent, such a co-agent may be formulated together with the compound of the present invention and administered in the form of the thus-obtained single formulation, or alternatively, such a co-agent may be formulated separately from the compound of the present invention and the co-agent and the compound of the present invention may be administered simultaneously or sequentially through an administration route that is the same as or different from the administration route of the pharmaceutical or agent for promoting expression of the present invention. The dose of such a co-agent may be set to an amount normally used when it is administered alone, or may be set smaller than the amount normally used.

When the agent for promoting expression of the present invention is administered to cells, administration can typically be performed by culturing the cells in a culture medium comprising the agent for promoting expression of the present invention. Such culture can be carried out under conditions commonly used in the art, as long as the expression of the FMR1 gene can be promoted under such conditions.

The present invention will be described more specifically with reference to the following examples. It is to be noted, however, that the present invention is not limited to these examples by any means.

### EXAMPLES

### Materials and Methods

### Generation of iPS cells and cell culture

According to a previously reported method, human iPS cells were generated from peripheral blood mononuclear cells (PBMCs) or human skin fibroblasts derived from FXS patients (FX1, FX2, and FX3) having expanded CGG repeats in the 5' UTR of the FMR1 gene and healthy individuals (HC1 and HC2) not having the expanded repeats using a lentiviral vector or episomal vector carrying OCT3/4, Sox2, Klf4, L-Myc, Lin28, and dominant-negative p53 or carrying OCT3/4, Sox2, Klf4, L-Myc, Lin28, and p53 shRNA (Table 1). The cells were subjected to maintenance culture in a feeder-free medium.

**[Table 1]**

| | HC1 | HC2 | HC3 | FX1 | FX2 | FX3 |
|---|---|---|---|---|---|---|
| Associated disease | Healthy control | Healthy control | Healthy control | FXS | FXS | FXS |
| Sex | Female | Male | Female | Male | Male | Male |
| Source | Human skin fibroblasts | PBMC | PBMC | PBMC | PBMC | PBMC |
| Reprogramming vector | Lentiviral vector | Episomal vector | Episomal vector | Episomal vector | Episomal vector | Episomal vector |
| CGG repeat length in FMR1 gene | 30 (normal) | 29 (normal) | 36 (normal) | 469 | 865 | 808 |

### Induction of neurons

Neurons were prepared according to the method described in International Patent Publication No. WO2014/148646. In brief, constructs expressing Neurogenin 2 under tetracycline induction were introduced into the iPS cells using a PiggyBac vector, whereby stable lines were generated. Doxycycline was added to the iPS cells, and the iPS cells were cultured for 7 days using a neuro-differentiation medium to generate neurons.

### Measurement of methylation in CpG island of FMR1 gene in iPS cells and neurons

Bisulfite sequencing was performed. Bisulfite treatment converts C to U, but not in the case where C is methylated. The target promoter region of the bisulfite-treated DNA was subjected to sequencing, whereby a methylated CpG island was identified. As a result, the iPS cells derived from the FX patients and the neurons generated therefrom showed almost 100% methylation, whereas the iPS cells derived from the healthy individuals and the neurons generated therefrom showed less than 20% methylation. This suggests that expansion of CGG repeats resulted in methylation of the FMR1 promoter region.

### Western blotting

The neurons (day 10) generated from the iPS cells were lysed in RIPA buffer and then subjected to SDS-PAGE. Thereafter, proteins were transferred onto a hydrophobic membrane, and the FMRP protein was detected using FMRP antibody.

### Measurement of the number of synaptic puncta

The neurons generated from the iPS cells were immunostained with Synapsin-1 antibody and βIII-tubulin antibody. Images were acquired using InCell 6000, and the number of Synapsin-1 positive puncta was quantified.

### Measurement of neuronal activity

The neurons generated from the iPS cells were cultured for one month on MEA electrode dishes, and their neuronal activity was detected. The detected neuronal activity is shown in the form of a raster plot.

### High-throughput screening

FIG. 5 illustrates the overview of the screening method. The neurons generated from the iPS cells of the FX patients were cultured in 96-well-plates, and on day 10, about 600 compounds were added thereto. On day 12, RNA was extracted, and qPCR was performed.

### Statistical analysis

All data are shown as mean ± standard error. Comparisons between groups were made using one-way or two-way ANOVA, followed by a post-hoc test using Scheffe's multiple comparison. p < 0.05 was considered statistically significant.

### Measurement of FMR1 gene expression level

The neurons generated from the iPS cells of the FX patients were cultured in 24-well plates, and the compounds were added thereto on day 10. On day 12, proteins were collected, and FMRP was quantified by ELISA.

### Example 1: Analysis of FMRP expression level in cells having expanded CGG repeats in FMR1 gene

The degree of methylation and the amount of FMRP in the CpG island of the FMR1 gene were measured in the iPS cells and the neurons. The results are shown in FIGs. 1 and 2. As can be seen in FIGs. 1 and 2, the iPS cells and neurons having expanded CGG repeats showed about 100% methylation in the CpG island, and FMRP was not detected in these iPS cells and neurons. On this account, screening was performed to search for substances that promote the expression of the FMR1 gene.

### Example 2: Analysis of phenotype of neurons having expanded CGG repeats in FMR1 gene

In order to analyze the phenotype of the neurons having expanded CGG repeats in the FMR1 gene, the number of synaptic puncta and the neuronal activity were measured. The results are shown in FIGs. 2 and 3. As can be seen in FIGs. 2 and 3, the neurons having expanded CGG repeats in the FMR1 gene had more synaptic puncta and exhibited higher neuronal activity than the controls. That is to say, the neurons having expanded CGG repeats in the FMR1 gene reflected the pathophysiology of the FXS patients.

### Example 3: Selection of candidate substances

Screening was performed to search for existing drugs that increase the expression level of the FMR1 gene using the neurons having expanded CGG repeats in the FMR1 gene. The screening result is shown in FIG. 5. The following two existing drugs were selected from the hit compounds. The expression level of the FMR1 gene is expressed as a relative value with the mRNA expression level thereof when a vehicle (DMSO) was administered being defined as 1.

### (1) Dexrazoxane hydrochloride

Relative expression level of the FMR1 gene: 368.2509661

### (2) Crizotinib

Relative expression level of the FMR1 gene: 727.3654052

### Example 4: Examination of concentration-dependent FMR1 gene expression promoting effect of dexrazoxane hydrochloride or crizotinib

Next, the present example examined whether dexrazoxane hydrochloride or crizotinib can promote the expression of the FMR1 gene in a concentration-dependent manner. The results are shown in FIG. 6. As can be seen in FIG. 6, dexrazoxane hydrochloride and crizotinib both could promote the expression of the FMR1 gene in a concentration-dependent manner.

The above results demonstrate that dexrazoxane hydrochloride and crizotinib can promote the expression of the FMR1 gene in a concentration dependent manner. Accordingly, it is strongly suggested that these compounds can exhibit a therapeutic or preventive effect on fragile X syndrome.

### INDUSTRIAL APPLICABILITY

Dexrazoxane or an analog thereof and crizotinib or an analog thereof are useful for the treatment or prevention of fragile X syndrome. Notably, drugs that have already been put on the market as pharmaceuticals for other diseases have potential for allowing rapid development of pharmaceuticals that can treat or prevent fragile X syndrome at low cost because clinical and non-clinical data on safety and other matters have been accumulated for these drugs and libraries of neighboring compounds already exist.

The present application claims priority based on Japanese Patent Application No. 2022-045557 filed in Japan (filing date: March 22, 2022), the disclosure of which is incorporated herein in its entirety by reference.

## Claims

1. A therapeutic or prophylactic medicament for fragile X syndrome, comprising:
a compound represented by the following formula (I) or (II), or a salt thereof: 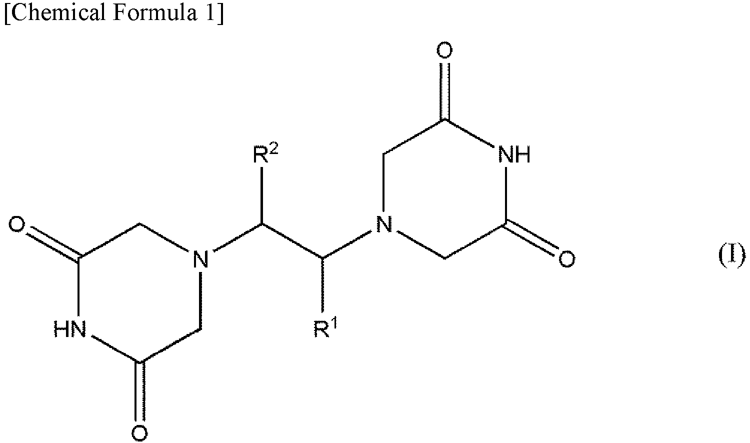 [in the formula (I),
R¹ and R² each independently represent a hydrogen atom or a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur; or R¹ and R² together may form a C₂-C₆ bridging group], 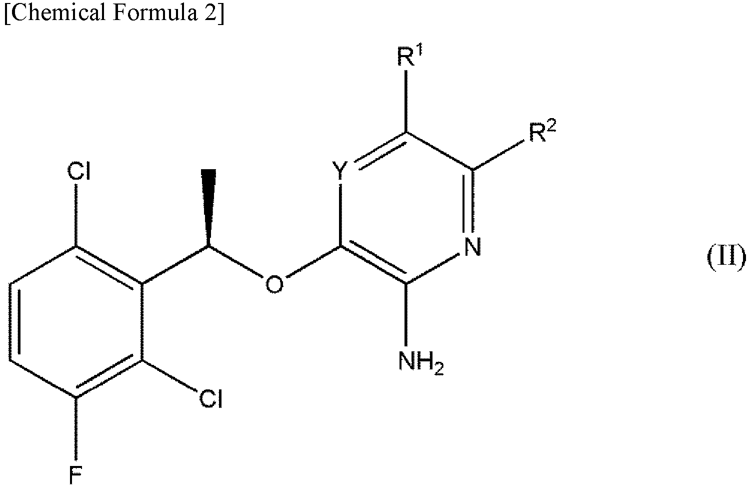 [in the formula (II),
Y represents a nitrogen atom or CZ [where Z is a hydrogen atom, a halogen atom, CN, or a C₁-C₃ aliphatic group in which each hydrogen atom may be substituted]; and
R¹ and R² each independently represent a hydrogen atom or a group that is selected from the group consisting of C₁-C₆ aliphatic groups, C₃-C₇ alicyclic hydrocarbon groups, and 5- to 10-membered monocyclic or bicyclic aromatic groups, wherein each hydrogen atom in the group may be substituted and the group may have 1 to 4 heteroatoms each independently selected from nitrogen, oxygen, and sulfur].

2. The therapeutic or prophylactic medicament according to claim 1, wherein R¹ and R² in the formula (I) are each independently a hydrogen atom or a C₁-C₃ aliphatic group in which each hydrogen atom may be substituted.

3. The therapeutic or prophylactic medicament according to claim 1 or 2, wherein the formula (I) is represented by the following formula (I-1): 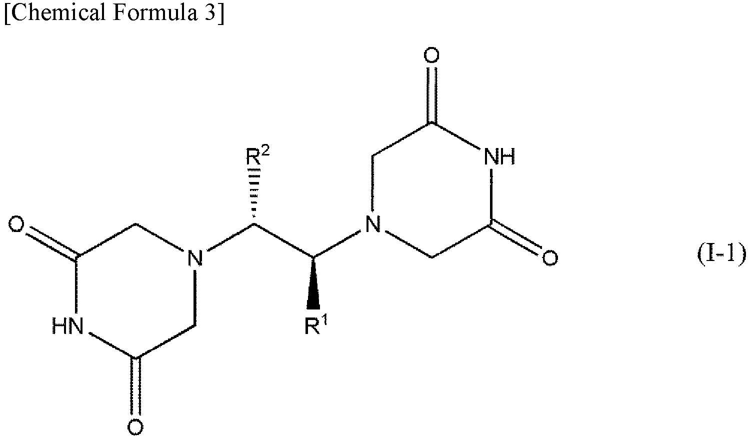 [in the formula (I-1), R¹ and R² each independently represent a hydrogen atom or a C₁-C₃ aliphatic group in which each hydrogen atom may be substituted].

4. The therapeutic or prophylactic medicament according to claim 3, wherein in the formula (I-1), R¹ is a methyl group and R² is a hydrogen atom.

5. The therapeutic or prophylactic medicament according to claim 1, wherein Y in the formula (II) is CZ [where Z represents a hydrogen atom, a halogen atom, or CN].

6. The therapeutic or prophylactic medicament according to claim 1 or 5, wherein R¹ in the formula (II) is a 5- to 10-membered monocyclic or bicyclic aromatic group in which each hydrogen atom may be substituted and that may have 1 to 4 heteroatoms each independently selected from nitrogen, oxygen, and sulfur.

7. The therapeutic or prophylactic medicament according to any one of claims 1, 5, and 6, wherein the formula (II) is the following formula (II-1):

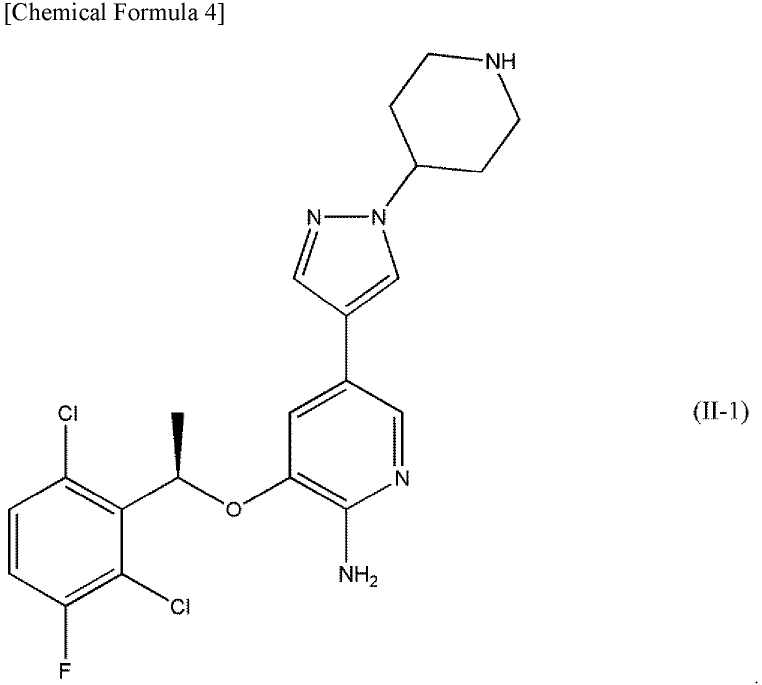

8. An agent for promoting FMR1 gene expression, comprising:
a compound represented by the following formula (I) or (II), or a salt thereof: 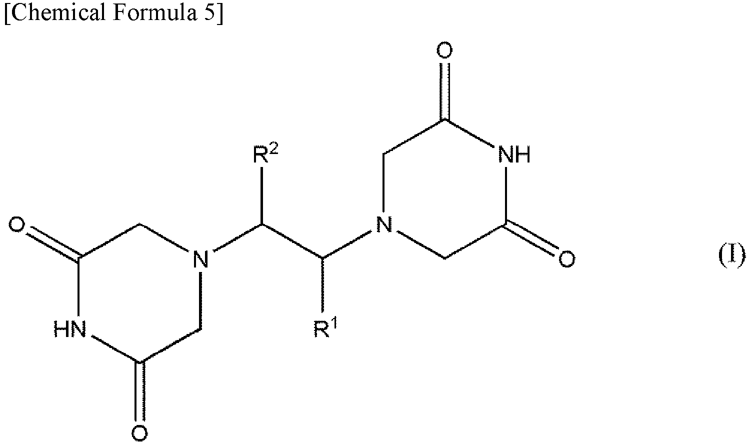 [in the formula (I),
R¹ and R² each independently represent a hydrogen atom or a group that is selected from the group consisting of C₁-C₆ aliphatic groups and C₃-C₇ alicyclic hydrocarbon groups, wherein each hydrogen atom in the group may be substituted and the group may have 1 to 4 heteroatoms each independently selected from nitrogen, oxygen, and sulfur; or R¹ and R² together may form a C₂-C₆ bridging group], 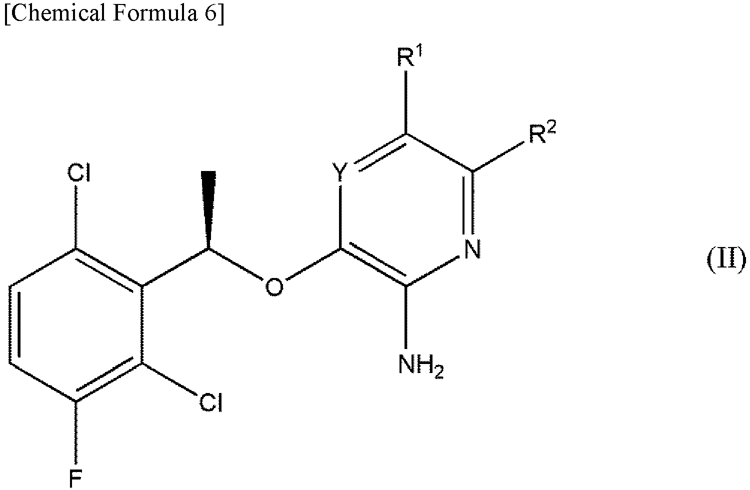 [in the formula (II),
Y represents a nitrogen atom or CZ [where Z is a hydrogen atom, a halogen atom, CN, or a C₁-C₃ aliphatic group in which each hydrogen atom may be substituted]; and
R¹ and R² each independently represent a hydrogen atom or a group that is selected from the group consisting of C₁-C₆ aliphatic groups, C₃-C₇ alicyclic hydrocarbon groups, and 5- to 10-membered monocyclic or bicyclic aromatic groups, wherein each hydrogen atom in the group may be substituted and the group may have 1 to 4 heteroatoms each independently selected from nitrogen, oxygen, and sulfur].
